# EUROPEAN PATENT SPECIFICATION

(11) **EP 2 142 005 B1**
(45) Date of publication and mention of the grant of the patent: **18.08.2010**
(21) Application number: 08735542.6
(22) Date of filing: 28.03.2008
(51) Int. Cl.: A23B 7/015, A23L 3/32, C12N 9/02, C12N 9/04, C12N 9/82, A23L 1/025, A23L 1/015

(54) **PROCESS FOR TREATING VEGETABLE MATERIAL WITH AN ENZYME**
VERFAHREN ZUR BEHANDLUNG VON PFLANZLICHEM MATERIAL MIT EINEM ENZYM
PROCÉDÉ DE TRAITEMENT D'UN MATÉRIAU VÉGÉTAL AVEC UNE ENZYME

(30) Priority: 29.03.2007 EP 07105190
(43) Date of publication of application: 13.01.2010
(73) Proprietor: Novozymes A/S, 2880 Bagsværd (DK)
(72) Inventor: KALUM, Lisbeth, 3500 Vaerloese (DK); HENDRIKSEN, Hanne Vang, 2840 Holte (DK)
(86) International application number: PCT/EP2008/053693
(87) International publication number: WO 2008/119749

(56) References cited:
- WO-A-2004/026043
- WO-A-2006/121397
- US-A1- 2005 211 638
- PRAPORSCIC ET AL: "Analysis of juice colour and dry matter content during pulsed electric field enhanced expression of soft plant tissues" JOURNAL OF FOOD ENGINEERING, BARKING, ESSEX, GB, vol. 79, no. 2, 1 November 2006 (2006-11-01), pages 662-670, XP005844975 ISSN: 0260-8774

## Description

### FIELD OF THE INVENTION

The present invention relates to a process for treating vegetable material with an enzyme.

### BACKGROUND OF THE INVENTION

Enzymes are sometimes used to treat vegetable material with intact cells where the substrate for the enzyme is present inside the cell membrane. One such example is enzymatic treatment of potato products.

It is known that acrylamide can be formed during deep frying of potatoes to make products such as potato chips and french-fried potatoes. It is known from WO 2004/026042, WO 2004/026043, WO 2004/030468, WO 2004/032648, and WO 2006/053563 that the acrylamide formation in such products may be reduced by a treatment with asparaginase or an oxidoreductase such as glucose oxidase to reduce the amount of asparagine or glucose in the potato product before the deep frying.

H.G.L. Coster, Biophysics Journal 5, 669-686 (1965) and E. Williams et al., Biophysics Journal, 8, 145-147 (1968) describe the possibility of using high intensity electric fields to permeabilize the cell membrane of vegetable materials.

WO 2006/121397 describes the use of electroporation at an electric field strength of 0.2-10 kV/cm for treating cellular potato material to produce holes (pores) in the cell membrane and make French fries, potato chips or potato crisps.

### SUMMARY OF THE INVENTION

The inventors have found that the enzymatic effect on an intracellular substrate present in vegetable cells with a membrane can be increased by pre-treating the vegetable material with a pulsed electric field.

Accordingly, the invention provides a process for treating vegetable material with an enzyme, comprising:
a) providing vegetable material comprising cells having a membrane and comprising an intracellular substrate for the enzyme,
b) treating the material with a pulsed electric field, and subsequently
c) treating the material with the enzyme.

### DETAILED DESCRIPTION OF THE INVENTION

### Vegetable material

The invention is applicable to enzymatic treatment of vegetable material comprising cells which have a membrane and which comprise an intracellular substrate for the enzyme. As an example, the cells may comprise intracellular asparagine and/or an intracellular reducing sugar.

Thus, the invention is applicable to an enzymatic pre-treatment of tubers such as potato (tubers from *Solanum tuberosum)* with the aim of reducing the level of acrylamide in food products made by heating (e.g. frying) of the potatoes, such as potato chips or french fries. The enzyme may be an enzyme capable of reacting on asparagine or an oxidoreductase capable of oxidizing the reducing sugar. Typical conditions for the enzymatic treatment are pH 4.5-8.5 and 25-60°C for 5-30 minutes.

Thus, one aspect of the invention provides a process, comprising the sequential steps of:
a) providing vegetable material comprising potato cells having a membrane,
b) treating the material with a pulsed electric field (PEF),
c) treating the material with an enzyme capable of reacting on asparagine or an oxidoreductase capable of oxidizing the reducing sugar, and
d) heating the material to make a food product.

The process typically comprises washing, peeling, and cutting (e.g. slicing) the potatoes. The process may further comprise parfrying, blanching, freezing and thawing, e.g. as described in WO 2006/05363. The food product may particularly be potato chips or French fries.

The enzyme treatment may be performed as described in the example below or in WO 2004/026042, WO 2004/026043, WO 2004/030468, WO 2004/032648, or WO 2006/053563. The treatment may be performed by incubating the tuber material in an aqueous enzyme solution. Alternatively, the tuber material may be sprayed with or immersed in such a solution, followed by incubation, e.g. during drying or transportation.

### Enzyme capable of reacting on asparagine

The enzyme capable of reacting with asparagine may be an asparaginase (EC 3.5.1.1), e.g. derived from *Aspergillus oryzae, Aspergillus nidulans, Aspergillus niger, Aspergillus fumigatus, Erwinia chrysanthemii, Saccharomyces cerevisiae, Candia utilis, Escherichia coli, Fusarium graminearum,* or *Penicillium citrinum,* e.g. as described in WO 2004/032648 or WO 2004/030468, such as the amino acid sequence shown in SEQ ID NO: 2 of WO 2004/032648.

The asparaginase may be used at a dosage of 200 to 100,000 ASNU per kg of vegetable solids, particularly 1,000-40,000 ASNU/kg, or 2,000-20,000 ASNU/kg. 1 ASNU (asparaginase unit) is defined as the amount of enzyme needed to generate 1.0 micromole of ammonia per minute at 37°C, pH 7.0 and a substrate concentration of 10 mg/mL.

### Oxidoreductase capable of reacting with a reducing sugar as a substrate

The oxidoreductase may be an oxidase or a dehydrogenase capable of reacting with a reducing sugar as a substrate such as glucose or maltose.

The oxidase may be a glucose oxidase, a pyranose oxidase, a hexose oxidase, a galactose oxidase (EC 1.1.3.9) or a carbohydrate oxidase which has a higher activity on maltose than on glucose. The glucose oxidase (EC 1.1.3.4) may be derived from *Aspergillus niger* e.g. having the amino acid sequence described in US 5094951. The hexose oxidase (EC 1.1.3.5) may be derived from algal species such as *Iridophycus flaccidum, Chondrus crispus* and *Euthora cristata.* The pyranose oxidase may be derived from *Basidiomycete* fungi, *Peniophora gigantean, Aphyllophorales, Phanerochaete chrysosporium, Polyporus pinsitus, Bierkandera adusta* or *Phlebiopsis gigantean.* The carbohydrate oxidase which has a higher activity on maltose than on glucose may be derived from *Microdochium* or *Acremonium,* e.g. from *M. nivale* (US 6165761), *A. strictum, A. fusidioides* or *A. potronii.*

The dehydrogenase may be glucose dehydrogenase (EC 1.1.1.47, EC 1.1.99.10), galactose dehydrogenase (EC 1.1.1.48), D-aldohexose dehydrogenase (EC 1.1.1.118, EC 1.1.1.119), cellobiose dehydrogenase (EC 1.1.5.1, e.g. from *Humicola insolens),* fructose dehydrogenase (EC 1.1.99.11, EC 1.1.1.124, EC 1.1.99.11), aldehyde dehydrogenase (EC 1.2.1.3, EC 1.2.1.4, EC 1.2.1.5). Another example is glucose-fructose oxidoreductase (EC 1.1.99.28).

The oxidoreductase is used in an amount which is effective to reduce the amount of acrylamide in the final product. For glucose oxidase, the amount may be in the range 50-20,000 (e.g. 100-10,000 or 1,000-5,000) GODU/kg dry matter in the raw material. One GODU is the amount of enzyme which forms 1 micromole of hydrogen peroxide per minute at 30°C, pH 5.6 (acetate buffer) with glucose 16.2 g/l (90 mM) as substrate using 20 min. incubation time. For other enzymes, the dosage may be found similarly by analyzing with the appropriate substrate.

### Pulsed electric field

The material with vegetable cells is treated with a pulsed electric field so as to create pores in the cell membranes, preferably resulting in an enhanced rate of mass transfer of intracellular substances. The electric field may have a field strength (voltage) above 10 kV/cm, above 20 kV/cm, or above 30 kV/cm, and preferably below 50 or below 40 kV/cm. The pulsed electric field may have a frequency of 10-200 pulses/min and duration of 0.5-5 minutes.

The electric field pulses may be applied in the form of exponential decaying, square-wave, oscillatory, bipolar, or instant reverse charges. The pulse width may be 2-50 microseconds. The electric field treatment may be performed continuously, e.g. as described in

### EXAMPLES

### Example 1: Evaluation of pulsed electric field as pretreatment for potato slices

### Procedure:

Bintje potatoes were peeled and sliced (1.4 mm). 400-450 ml tap water was added to 300 g potato slices and transferred to the treatment chamber (total volume 750 ml). Four different field strengths (0, 10, 20 and 35 kV/cm) with 100 pulses over 2 min. were applied. After the PEF treatment, the potato slices and tap water were transferred to a beaker glass and incubated with or without asparaginase (31500 U/I) for 20 min at room temperature. Water samples and potato slices were frozen. The frozen potato slices (without thawing) were deep fried for 210 seconds in vegetable oil at 180°C.

Monosaccharide (glucose) in water samples was analyzed with a blood sugar device immediately after enzyme incubation. Amino acids were analyzed using HPLC. Texture was evaluated qualitatively after the PEF treatment. Acrylamide in the fried product was determined by HPLC after extraction, and the dry substance content was determined by drying at 105°C for 40 hours.

The results are shown in the following table (bq = below quantification = 0.013 mM; bd = below detection; enz = asparaginase; Asp = aspartic acid; Asn = asparagine):

| | | | | | | | | | | | |
|---|---|---|---|---|---|---|---|---|---|---|---|
| Sample treatment | Glucose (mM) | | Amino acid (mM) No Enz | | Amino acid (mM) Enz | | DS after frying | | Acrylamide (ppm) after frying | | Texture |
| | No enz | enz | Asp | Asn | Asp | Asn | No enz | enz | No enz | enz | |
| 0 kV/cm | bq | bq | bq | bq | 0.04 | bd | 95.6 | 93.9 | 19054 | 4571 | Crisp |
| 10 kV/cm | 5.9 | 8.0 | bq | 0.19 | 0.24 | bd | 94.9 | 95.0 | 18673 | 4795 | Slightly crisp |
| 20 kV/cm | 6.9 | 7.4 | bq | 0.09 | 0.31 | bd | 92.6 | 94.8 | 9456 | 3864 | Soft |
| 35 kV/cm | 8.0 | 7.3 | 0.03 | 0.23 | 0.28 | bd | 95.2 | 93.9 | 16354 | 2700 | Very soft |

Enhanced leaching of glucose was observed at field strength level above 10 kV/cm. Tissue softening was found to increase with increasing field strength changing from crisp (no PEF) to very soft (35 kV/cm). Acrylamide level was almost not affected by the PEF treatment alone. Asparaginase was found to reduce the overall level significantly. A synergistic effect was observed when combining asparaginase with high field strength, above 20 kV/cm.

## Claims

1. A process for treating vegetable material with an enzyme, comprising:
a) providing vegetable material comprising cells having a membrane and comprising an intracellular substrate for the enzyme,
b) treating the material with a pulsed electric field, and subsequently
c) treating the material with the enzyme.

2. The process of the preceding claim wherein:
a) the cells comprise intracellular asparagine and/or an intracellular reducing sugar,
b) the enzyme is an enzyme capable of reacting on asparagine or an oxidoreductase capable of oxidizing the reducing sugar, and
c) the process comprises heat treatment after the enzyme treatment.

3. The process of the preceding claim wherein the enzyme is an asparaginase.

4. The process of a preceding claim wherein the cells comprise intracellular glucose.

5. The process of the preceding claim wherein the enzyme is glucose oxidase, hexose oxidase or pyranose oxidase.

6. The process of a preceding claim wherein the vegetable material comprises pieces of tuber, particularly potato.

7. The process of the preceding claim, comprising the sequential steps of:
a) providing vegetable material comprising potato cells having a membrane,
b) treating the material with a pulsed electric field,
c) treating the material with an enzyme capable of reacting on asparagine or an oxidoreductase capable of oxidizing the reducing sugar, and
d) heating the material to make a food product.

8. The process of the preceding claim wherein the heating comprises frying, and the food product is potato chips or French fries.

9. The process of any preceding claim wherein the pulsed electric field has a field strength above 20 kV/cm, particularly above 30 kV/cm.

10. The process of any preceding claim wherein the pulsed electric field has a frequency of 10-200 pulses/min and duration of 0.5-5 minutes.

## Patentansprüche

1. Verfahren zum Behandeln von Pflanzenmaterial mit einem Enzym, umfassend:
a) Bereitstellen von Pflanzenmaterial, umfassend Zellen mit einer Membran und umfassend ein intrazelluläres Substrat fiir das Enzym,
b) Behandeln des Materials mit einem gepulsten elektrischen Feld, und anschließend
c) Behandeln des Materials mit dem Enzym.

2. Verfahren nach dem vorhergehenden Anspruch, wobei:
a) die Zellen intrazelluläres Asparagin und/oder einen intrazellulären reduzierenden Zucker umfassen,
b) das Enzym ein zum Umsetzen von Asparagin fähiges Enzym ist oder eine zum Oxidieren des reduzierenden Zuckers fähige Oxidoreduktase ist, und
c) das Verfahren Hitzebehandlung nach der Enzymbehandlung umfasst.

3. Verfahren nach dem vorhergehenden Anspruch, wobei das Enzym eine Asparaginase ist.

4. Verfahren nach einem vorhergehenden Anspruch, wobei die Zellen intrazelluläre Glucose umfassen.

5. Verfahren nach dem vorhergehenden Anspruch, wobei das Enzym Glucoseoxidase, Hexoseoxidase oder Pyranoseoxidase ist.

6. Verfahren nach einem vorhergehenden Anspruch, wobei das Pflanzenmaterial Knollenstücke umfasst, insbesondere Kartoffel.

7. Verfahren nach dem vorhergehenden Anspruch, umfassend die sequentiellen Schritte von:
a) Bereitstellen von Pflanzenmaterial, umfassend Kartoffelzellen mit einer Membran,
b) Behandeln des Materials mit einem gepulsten elektrischen Feld,
c) Behandeln des Materials mit einem zum Umsetzen von Asparagin fähigen Enzym oder mit einer zum Oxidieren des reduzierenden Zuckers fähigen Oxidoreduktase, und
d) Erhitzen des Materials, zur Herstellung eines Lebensmittelproduktes.

8. Verfahren nach dem vorhergehenden Anspruch, wobei das Erhitzen Frittieren umfasst, und das Lebensmittelprodukt Kartoffelchips oder Pommes Frites sind.

9. Verfahren nach einem beliebigen vorhergehenden Anspruch, wobei das gepulste elektrische Feld eine Feldstärke von oberhalb 20 kV/cm, insbesondere oberhalb 30 kV/cm hat.

10. Verfahren nach einem beliebigen vorhergehenden Anspruch, wobei das gepulste elektrische Feld eine Frequenz von 10-200 Pulsen/min und eine Dauer von 0,5-5 Minuten hat.

## Revendications

1. Méthode de traitement de matériel végétal avec une enzyme, comprenant :
a) la fourniture d'un matériel végétal comprenant des cellules ayant une membrane et comprenant un substrat intracellulaire pour l'enzyme,
b) le traitement du matériel avec un champ électrique pulsé, et par la suite
c) le traitement du matériel avec l'enzyme.

2. Méthode de la revendication précédente dans laquelle :
a) les cellules comprennent de l'asparagine intracellulaire et/ou un sucre réducteur intracellulaire,
b) l'enzyme est une enzyme capable de réagir avec l'asparagine ou une oxydoréductase capable d'oxyder le sucre réducteur, et
c) la méthode comprend un traitement par la chaleur après le traitement avec l'enzyme.

3. Méthode de la revendication précédente dans laquelle l'enzyme est une asparaginase.

4. Méthode d'une revendication précédente dans laquelle les cellules comprennent du glucose intracellulaire.

5. Méthode de la revendication précédente dans laquelle l'enzyme est une glucose oxydase, une hexose oxydase ou une pyranose oxydase.

6. Méthode d'une revendication précédente dans laquelle le matériel végétal comprend des morceaux de tubercules, particulièrement de pommes de terre.

7. Méthode de la revendication précédente, comprenant les étapes séquentielles consistant à :
a) fournir du matériel végétal comprenant des cellules de pommes de terre ayant une membrane,
b) traiter le matériel avec un champ électrique pulsé,
c) traiter le matériel avec une enzyme capable de réagir avec une asparagine ou une oxydoréductase capable d'oxyder le sucre réducteur, et
d) chauffer le matériel pour faire un produit alimentaire.

8. Méthode de la revendication précédente dans laquelle le chauffage comprend la friture, et le produit alimentaire est des chips de pommes de terre ou des frites.

9. Méthode de l'une quelconque des revendications précédentes dans laquelle le champ électrique pulsé a une force de champ supérieure à 20 kV/cm, particulièrement supérieure à 30 kV/cm.

10. Méthode de l'une quelconque des revendications précédentes dans laquelle le champ électrique pulsé a une fréquence de 10-200 pulsations/min et une durée de 0,5-5 minutes.
